# EUROPEAN PATENT SPECIFICATION

(11) **EP 4 417 224 B1**
(45) Date of publication and mention of the grant of the patent: **24.06.2026**
(21) Application number: 22880141.1
(22) Date of filing: 27.09.2022
(51) Int. Cl.: A61L 27/18, A61L 27/20, A61L 27/26, A61L 27/50, B01J 3/04

(54) **INJECTABLE POLYHYDROXYALKANOATE MICROSPHERES AND PREPARATION METHOD THEREFOR**
INJIZIERBARE POLYHYDROXYALKANOATMIKROKUGELN UND HERSTELLUNGSVERFAHREN DAFÜR
MICROSPHÈRES DE POLYHYDROXYALCANOATE INJECTABLES ET LEUR PROCÉDÉ DE PRÉPARATION

(30) Priority: 12.10.2021 CN 202111187296
(43) Date of publication of application: 21.08.2024
(73) Proprietor: Beijing Joyinera Biomaterial Technology Co., Ltd., Beijing 102206 (CN)
(72) Inventor: WEI, Daixu, 102206 Beijing (CN); LI, Teng, 102206 Beijing (CN); ZHANG, Haoqian, 102206 Beijing (CN); TAN, Chang, 102206 Beijing (CN)
(74) Representative: Grünecker Patent- und Rechtsanwälte PartG mbB
(86) International application number: PCT/CN2022/121527
(87) International publication number: WO 2023/061201

(56) References cited:
- WO-A1-2023/197419
- CN-A- 104 667 346
- CN-A- 110 200 936
- CN-A- 113 230 451
- CN-A- 113 274 954
- CN-A- 113 616 604
- CN-A- 114 588 310
- CN-A- 114 788 895
- RU-C1- 2 692 768
- US-A1- 2003 194 443
- ZHANG JUNYU; SHISHATSKAYA EKATERINA I.; VOLOVA TATIANA G.; DA SILVA LUIZIANA FERREIRA; CHEN GUO-QIANG: "Polyhydroxyalkanoates (PHA) for therapeutic applications", MATERIALS SCIENCE AND ENGINEERING C, ELSEVIER SCIENCE S.A., CH, vol. 86, 5 January 2018 (2018-01-05), CH , pages 144 - 150, XP085358296, ISSN: 0928-4931, DOI: 10.1016/j.msec.2017.12.035

## Description

### Technical field

The invention belongs to the biomedical field, in particular relates to an injectable polyhydroxyalkanoate microsphere and a preparation method thereof.

### Background

As people get older or are affected by some diseases, the muscle and collagen tissue in the human body will have different degrees of functional degradation, resulting in skin depression, gastric reflux and other problems. Therefore, people have invented a variety of fillers to fill the depressed skin or stimulate the regeneration of muscle and collagen through foreign substances, such as hyaluronic acid, bovine collagen, etc. However, the filling effect of these substances as fillers lasts for a short period and it requires frequent reinjection to maintain their filling effect. In order to achieve long-term filling effect, people try to make microspheres by using non-biodegradable materials as filling agents, such as polyvinyl alcohol (PVA) and poly (methyl methacrylate) (PMMA), and the like. Although the filling effect of these fillers can be maintained with significantly increased period, when these materials remain in the body for too long, they will release harmful substances, thus causing a series of side effects and being harmful to human health.

In recent years, biodegradable polymer materials have come into public attentions. This type of material is non-toxic and non-repellent to human body, and can be gradually degraded with human metabolism and then discharged from the body. Moreover, the degradation time can vary from one week to several years by adjusting the molecular weight and other parameters of the material.

Polyhydroxyalkanoate, or PHA for short, is a natural polymer biomaterial and an intracellular polyester synthesized by microorganisms. Because of its good biocompatibility and biodegradability, PHA is one of the most ideal biomedical materials at present. PHA has good cell compatibility with cells in vivo, cells can grow well on this scaffold, and the scaffold can be degraded into CO₂ and H₂O. For the convenience of injection, it is usually made into microspheres, which can be injected through a needle. Because of the existence of human phagocytes, microspheres usually have a diameter of 20 microns or more, and too large microspheres may block the needle and even cause skin rupture. Therefore, the size of microspheres used for injection is usually 60 microns or less.

In the prior art, when injectable microspheres are prepared by using PHA material, aqueous polyvinyl alcohol (PVA) solution is usually used as the aqueous phase, and the emulsification method or microfluidic method is used for the preparation of microspheres. However, the microspheres produced by this process are prone to agglomerate and adhere, forming large blocky substances, which brings inconvenience to the subsequent injection, and greatly hinders the application of biodegradable material microspheres as fillers. Therefore, a novel microsphere preparation solution is urgently needed to solve the problems of microsphere aggregation and adhesion.

CN113230451 discloses an injectable dermal filler and a preparation method for preparing the injectable dermal filler.

### Summary of the invention

In view of the above, the purpose of the invention is to provide an injectable polyhydroxyalkanoate microsphere and a preparation method thereof. The preparation method provided by the invention can effectively improve the dispersion of polyhydroxyalkanoate microspheres and overcome the problems of microsphere aggregation and adhesion; at the same time, it can effectively improve the absorbability and injectability of polyhydroxyalkanoate microspheres.

The invention provides a preparation method of injectable polyhydroxyalkanoate microspheres, including the following steps:
a) dissolving a PHA material in an organic solvent to obtain an oil phase;
b) dissolving hyaluronic acid in water to obtain an aqueous phase;
c) adding the oil phase to the aqueous phase dropwise with stirring, thereafter evaporating off the organic solvent under continuous stirring, and then carrying out solid-liquid separation and drying to obtain PHA microspheres; the order of steps a) and b) is not limited.

Preferably, the hyaluronic acid has a molecular weight of 5-500KDa.

Preferably, the mass concentration of hyaluronic acid in the aqueous phase is 0.01% -1% (w/v).

Preferably, in step c), the volume ratio of the oil phase to the aqueous phase is 1 : (10-200).

Preferably, the mass concentration of PHA material in the oil phase is 2.5%-10% (w/v);

The organic solvent is selected from one or more of dichloromethane, chloroform and ethyl acetate.

Preferably, the PHA material has a molecular weight of 10-100KDa.

Preferably, the PHA material is natural or unnatural polyhydroxyalkanoate;

The natural or non-natural polyhydroxyalkanoate includes but is not limited to one or more of PHB, PHBV, PHBHHx, P34HB, PHBVHHx, PHHx and PHO.

Preferably, the stirring rate is 100~500 rpm.

Preferably, the stirring is performed by using a magnetic stirrer; after the addition of the oil phase, the stirring time is ≥ 4h.

The invention also provides an injectable polyhydroxyalkanoate microsphere prepared by the preparation method described in the above technical solutions.

In the preparation method provided by the invention, the PHA material is dissolved in an organic solvent to prepare an oil phase, a hyaluronic acid aqueous solution is used as an aqueous phase, the aqueous phase is stirred and the oil phase is slowly added into the aqueous phase, and then the organic solvent evaporates off under continuous stirring, and then the solid-liquid separation and drying are carried out to prepare the PHA microspheres. In the above method of the invention, the dispersion of PHA microspheres can be improved, and the resulting microspheres can be rapidly dispersed in water and the microspheres are complete in morphology, independent of each other, and there is no agglomeration phenomenon, so the needle can be prevented from being blocked and subsequent injection of the PHA microspheres as a filler is facilitated. At the same time, the polyhydroxyalkanoate microspheres prepared by the invention have good absorbability and injectability. Moreover, PHA microspheres have excellent biocompatibility and will not cause rejection reaction, and can achieve long-term filling effect.

The experimental results show that the preparation method provided by the invention has the following beneficial effects: ① the yield of microspheres is high: the yield of microspheres is 70% or more. ② Good dispersibility: The PHA microspheres prepared by the invention can be dispersed in water upon proper mixing. After standing, the microspheres show good dispersibility in water and no layering, they neither float on the upper layer of water nor settle on the bottom of water, rather they are evenly dispersed in water to form a uniform dispersion. The microspheres are observed for their morphology after the above dispersion is air dried and the results show that the microspheres are complete in morphology, independent of each other, and there is no agglomeration phenomenon, all or most of the microspheres have particle size of 60 µm or less, and they can meet the injection requirements. ③ Good absorbability: The microspheres have an absorption rate of 85% or above by using a syringe to draw the above dispersion, showing excellent absorbability. ④ Good injectability: Pushing a syringe and collecting the dispersion injected out, calculating the injection rate of the microspheres, and the results show that the injection rate of the microspheres is 88% or above, showing excellent injectability.

### BRIEF DESCRIPTION OF THE DRAWINGS

In order to more clearly explain the technical solutions in the embodiments of the invention or in the prior art, the drawings needed in the description of the embodiments or the prior art will be briefly introduced as below. It is obvious that the drawings in the following description are only examples of the invention. For a person skilled in the art, other drawings can also be obtained according to the provided drawings without any creative work.
Figure 1 is an optical microscope picture of injectable PHBHHx microspheres obtained in Example 1;
Figure 2 is an optical microscope picture of injectable PHBVHHx microspheres obtained in Example 2;
Figure 3 is an optical microscope picture of injectable PHB microspheres obtained in Example 3;
Figure 4 is an optical microscope picture of injectable PHBV microspheres obtained in Example 4;
Figure 5 is an optical microscope picture of injectable P34HB microspheres obtained in Example 5;
Figure 6 is a schematic diagram showing the dispersion stability of microspheres obtained in examples 1-5 and comparative example 1-5.

### EMBODIMENTS

The invention provides a preparation method of injectable polyhydroxyalkanoate microspheres, including the following steps:
a) Dissolving a PHA material in an organic solvent to obtain an oil phase;
b) Dissolving hyaluronic acid in water to obtain an aqueous phase;
c) Adding the oil phase to the aqueous phase dropwise with stirring, thereafter evaporating off the organic solvent under continuous stirring, and then carrying out solid-liquid separation and drying to obtain PHA microspheres;

The order of steps a) and b) is not limited.

In the preparation method provided by the invention, the PHA material is dissolved in an organic solvent to obtain an oil phase, a hyaluronic acid aqueous solution is used as an aqueous phase, the aqueous phase is stirred and the oil phase is slowly added into the aqueous phase, and then the organic solvent evaporates off under continuous stirring, and then the solid-liquid separation and drying are carried out to prepare the PHA microspheres. In the above method of the invention, the dispersion of PHA microspheres can be improved, and the resulting microspheres can be rapidly dispersed in water and the microspheres are complete in morphology, independent of each other, and there is no agglomeration phenomenon, so the needle can be prevented from being blocked and subsequent injection of the PHA microspheres as a filler is facilitated. At the same time, the polyhydroxyalkanoate microspheres prepared by the invention have good absorbability and injectability. Moreover, the PHA microspheres have excellent biocompatibility and will not cause rejection reaction, and can achieve long-term filling effect.

**For step a):** Dissolving a PHA material in an organic solvent to obtain an oil phase. In the present invention, the type of PHA material (i.e. polyhydroxyalkanoate) is not limited, as long as it is a conventional five-generation PHA commercial product in the field, specifically, natural or non-natural polyhydroxyalkanoate; More specifically, one or more of PHB (i.e. 3-hydroxybutyrate), PHBV (i.e. copolymer of hydroxybutyrate and hydroxyvalerate), PHBHHx (i.e. P3HB-co-3HHx, copolyester of 3-hydroxybutyric acid and 3-hydroxycaproic acid), P34HB (i.e. P3HB-co-4HB, copolyester of 3-hydroxybutyric acid and 4-hydroxybutyrate), PHBVHx (i.e. copolymer of 3-hydroxybutyric acid, 3-hydroxycaproic acid and 3-hydroxyvaleric acid), PHHx (i.e. polyhydroxycaproate) and PHO (i.e. polyhydroxy octanoate). In the invention, the molecular weight of the PHA material is preferably 10-100KDa; In some embodiments of the invention, the molecular weight is 10-30 KDa, 20-100 KDa, or 10-100 KDa. The average molecular weight of the PHA material is preferably 15-80 KDa. In some embodiments of the invention, the average molecular weight is 15 KDa, 40 KDa, 50 KDa or 80 KDa.

In some embodiments of the invention, the PHA material is PHBHHx, with a molecular weight of 20-100 KDa and an average molecular weight of 50 KDa. In other embodiments of the invention, the PHA material is PHBVHHx, with a molecular weight of 20-100 KDa and an average molecular weight of 50 KDa. In other embodiments of the invention, the PHA material is PHB, with a molecular weight of 20-100 KDa and an average molecular weight of 80 KDa. In other embodiments of the invention, the PHA material is PHBV, with a molecular weight of 20-100 KDa and an average molecular weight of 40 KDa. In other embodiments of the invention, the PHA material is P34HB, with a molecular weight of 10-30 KDa and an average molecular weight of 15 KDa.

In the invention, the organic solvent is preferably one or more of dichloromethane, chloroform and ethyl acetate. The invention has no special restriction on the source of the organic solvents, as long as they are commercially available.

In the invention, the mass concentration (w/v) of the organic solution which is formed by dissolving the PHA material in an organic solvent is preferably 2.5%-10%; In some embodiments of the invention, the mass concentration is 2.5%, 5% or 10%. In the present application, the organic solution formed by dissolving PHA material in an organic solvent is used as oil phase.

**For step** b): Dissolving hyaluronic acid in water to obtain an aqueous phase.

In the invention, the molecular weight of the hyaluronic acid (i.e. HA) is preferably 5-500KDa; In some embodiments of the invention, the molecular weight is 5-100 KDa, 10-200 KDa or 100-500 KDa. In the invention, the average molecular weight of the hyaluronic acid is preferably 50-300 KDa. In some embodiments of the invention, the average molecular weight is 50KDa or 300KDa.

In some embodiments of the invention, the hyaluronic acid has a molecular weight of 5-100 KDa, and an average molecular weight of 50 KDa. In other embodiments of the invention, the hyaluronic acid has a molecular weight of 10-200 KDa, and an average molecular weight of 50 KDa. In other embodiments of the invention, the hyaluronic acid has a molecular weight of 100-500 KDa, and an average molecular weight of 300 KDa.

In the present invention, the water is preferably distilled water or deionized water, more preferably distilled water.

In the invention, the mass concentration (w/v) of the aqueous solution formed by dissolving the hyaluronic acid in water is preferably 0.01%-1%; In some embodiments of the invention, the mass concentration is 0.5%, 5% or 10%. In the present invention, the aqueous solution formed by dissolving hyaluronic acid in water is used as the aqueous phase. In the prior art, polyvinyl alcohol (PVA) aqueous solution is usually used as the aqueous phase to prepare PHA microspheres, and the resulting PHA microspheres are prone to agglomerate and adhere in water, and are difficult to disperse quickly, whereas the present invention uses hyaluronic acid aqueous solution as the aqueous phase, which can improve the dispersion of PHA microspheres, and the resulting microspheres can be rapidly dispersed in water and the microspheres are complete in morphology, independent of each other, and there is no agglomeration phenomenon, thus the needle can be prevented from being blocked, and subsequent injection of the PHA microspheres as a filler is facilitated. At the same time, the polyhydroxyalkanoate microspheres prepared by the invention have good absorbability and injectability. Moreover, PHA microspheres have excellent biocompatibility and will not cause rejection reaction, and can achieve long-term filling effect.

In the invention, the order of the above steps a) and b) is not limited.

**For step c):** Adding the oil phase to the aqueous phase dropwise with stirring, thereafter evaporating off the organic solvent under continuous stirring, and then carrying out solid-liquid separation and drying to obtain PHA microspheres.

In the present invention, after the oil phase and aqueous phase are obtained, the aqueous phase is stirred, and the oil phase is slowly added dropwise to the aqueous phase with the stirring. Wherein, the stirring is performed by using a magnetic stirrer. The stirring rate is 100-500rpm, typically, the stirring rate can be but not limited to 100rpm, 200rpm, 300rpm, 400rpm or 500rpm. The volume ratio of the oil phase to the aqueous phase is preferably 1 : (10-200); In some embodiments of the invention, the volume ratio is 1 : 10, 1 : 20, 1 : 50, 1 : 100, 1 : 150 or 1 : 200.

After the addition of all the oil phase, the stirring process preferably includes: first continue to stir to form emulsion, and then continue to stir to volatilize the organic solvent. In the continuous stirring process, the stirring is performed by using a magnetic stirrer. The stirring rate is 100-500 rpm; In some embodiments of the invention, the stirring rate is 400 rpm. In the continuous stirring process, the stirring is performed by using a magnetic stirrer; The stirring rate is 100-500rpm, typically, the stirring rate can be but not limited to 100rpm, 200rpm, 300rpm, 400rpm or 500rpm; The time of the continuous stirring is preferably ≥ 4h, more preferably 4-6h. In some embodiments of the invention, the time of continuous stirring (for volatilizing organic solvent) after the formation of emulsion is 4h, 5h or 6h. In the invention, the temperatures, at which the above adding oil phase and stirring are performed, are not limited, and they can be carried out at room temperature, specifically 10-37 °C, preferably 25 °C.

After the solvent is completely volatilized under continuous stirring, solid-liquid separation is carried out. The invention has no special restrictions on the manners of solid-liquid separation, which can be carried out according to the conventional method known to those skilled in the art, such as filtration.

After solid-liquid separation, water is removed by drying. In the present invention, preferably, the drying is performed by freezing first and then freeze-drying. In the invention, the drying temperature is preferably -80~-20°C; wherein, the freezing temperature is preferably -80~-20°C, and the freeze-drying temperature is preferably -60~-40°C. In some embodiments of the invention, the freezing temperature is -80~-40 °C and the time is 6~12h; the freeze-drying temperature is preferably -40 °C and the time is 24 h. After the above drying treatment, PHA microspheres are obtained.

The invention also provides a PHA microsphere prepared by the preparation method described in the above technical solution.

In the preparation method provided by the invention, the PHA material is dissolved in an organic solvent to prepare an oil phase, a hyaluronic acid aqueous solution is used as an aqueous phase, the aqueous phase is stirred and the oil phase is slowly added into the aqueous phase, and then the organic solvent evaporates off under continuous stirring, and then the solid-liquid separation and drying are carried out to prepare the PHA microspheres. In the above method of the invention, the dispersion of PHA microspheres can be improved, and the resulting microspheres can be rapidly dispersed in water and the microspheres are complete in morphology, independent of each other, and there is no agglomeration phenomenon, so the needle can be prevented from being blocked and subsequent injection of the PHA microspheres as a filler is facilitated. At the same time, the polyhydroxyalkanoate microspheres prepared by the invention have good absorbability and injectability. Moreover, PHA microspheres have excellent biocompatibility and will not cause rejection reaction, and can achieve long-term filling effect.

The experimental results show that:
(1) The preparation method provided by the invention has a microsphere yield of 70% or more. The PHA microspheres prepared by the invention can be dispersed in water upon proper mixing. After standing, the microspheres show good dispersibility in water and no layering, they neither float on the upper layer of the water nor settle on the bottom of the water, rather they are evenly dispersed in water to form a uniform dispersion. The microspheres are observed for their morphology after the above dispersion is air dried and the results show that the microspheres are complete in morphology, independent of each other, and there is no agglomeration phenomenon. All or most of the microspheres have particle size of 60 µm or less, and can meet the injection requirements.
(2) A disposable 1mL syringe with a needle with an inner diameter of 0.5mm and a length of 19.7mm (that is, the commonly used 1mL syringe gun head) is used as the device for detecting the absorption rate, and 1mL of microsphere dispersion (the dry weight of microsphere is 0.1g, marked as M0) is used as the experimental sample. All microsphere dispersions are drawn at one time. The microsphere that can be drawn into the syringe tube is withdrawn and freeze-dried, the mass of which is M1, and the absorption rate is defined as (M1/M0) × 100%_{∘} The above syringe is used to draw the above microsphere dispersion, and the microsphere absorption rate of the microspheres reaches 85% or more, showing excellent absorbability.
(3) A disposable 1mL syringe with a needle with an inner diameter of 0.5mm and a length of 19.7mm (that is, the commonly used 1mL syringe gun head) is used as the device for detecting the injection rate, and 1mL of microsphere dispersion (the dry weight of microsphere is 0.1g, marked as M0) is used as the experimental sample. 1mL of microsphere dispersion is injected into the syringe tube from the rear of the syringe, all microsphere dispersion is injected out at one time, the microspheres that can be injected into the outside of the syringe tube through the needle are collected and freeze-dried, the mass of which is M2, and the injection rate is defined as (M2/M0) × 100%. Pushing the syringe and collecting the dispersion injected out, calculating the injection rate of the microspheres, and the results show that the injection rate of the microspheres is 88% or more, showing excellent injectability.

In order to further understand the invention, the preferrable embodiments of the invention are described with reference to the examples below. However, it should be understood that these descriptions are only for further explaining the features and advantages of the invention, and are not intended to limit the claims of the invention.

### Example 1

1g of PHBHHx (molecular weight 20-100 KDa, average molecular weight 50 KDa) was dissolved in 20mL of dichloromethane to prepare an organic solution with a final concentration of 5% (w/v), which was the oil phase. Hyaluronic acid (molecular weight 5-100 KDa, average molecular weight 50 KDa) was dissolved in distilled water to prepare an aqueous solution with a final concentration of 0.5% (w/v), which was the aqueous phase. The aqueous phase was stirred using a magnetic stirrer at a speed of 400 rpm. At the same time, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 5h to remove the organic solvent. Thereafter, the obtained solution was first frozen (- 80 °C for 6h), and then freeze-dried (- 40 °C for 24h) to remove water to obtain injectable PHBHHx microspheres.

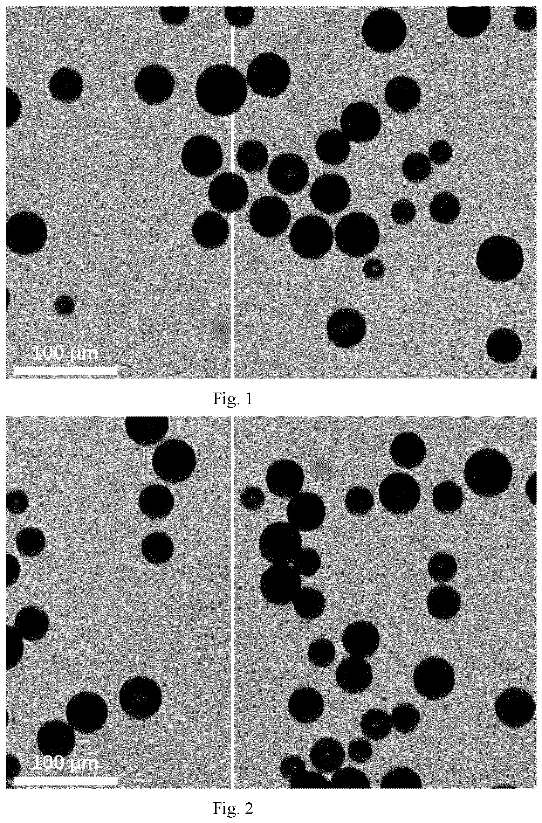

According to the input amount of PHBHHx material and the amount of PHBHHx microspheres obtained, the yield of injectable PHBHHx microspheres was calculated as 93%. The freeze-dried PHBHHx microspheres were dissolved in water, and they can be dispersed in water upon proper mixing. After the water was air dried, the resultant was observed on an optical microscope. The results are shown in Figure 1. Figure 1 is the optical microscope picture of the injectable PHBHHx microspheres obtained in Example 1. It can be seen that the obtained microspheres have good dispersion, which are spherical or ellipsoidal. The particle size is 1-50 µm as detected by particle size meter.

### Example 2

1g of PHBVHHx (molecular weight 20-100 KDa, average molecular weight 50 KDa) was dissolved in 20mL of dichloromethane to prepare an organic solution with a final concentration of 5% (w/v), which was the oil phase. Hyaluronic acid (molecular weight 10-200 KDa, average molecular weight 50 KDa) was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 4h to remove the organic solvent. Thereafter, the obtained solution was first frozen (- 40 °C for 12h), and then freeze-dried (- 40 °C for 24h) to remove water to obtain injectable PHBVHHx microspheres.

The yield of the obtained injectable PHBVHHx microspheres was 91%. The freeze-dried PHBVHHx microspheres were dissolved in water, and they can be dispersed in water upon proper mixing. After the water was air dried, the resultant was observed on an optical microscope. The results are shown in Figure 2. Figure 2 is the optical microscope picture of the injectable PHBVHHx microspheres obtained in Example 2. It can be seen that the obtained microspheres have good dispersion, which are spherical or ellipsoidal. The particle size is 4-50 µm as detected by particle size meter.

### Example 3

1g of PHB (molecular weight 20-100 KDa, average molecular weight 80 KDa) was dissolved in 20mL of dichloromethane to prepare an organic solution with a final concentration of 5% (w/v), which was the oil phase. Hyaluronic acid (molecular weight 100-500 KDa, average molecular weight 300 KDa) was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 6h to remove the organic solvent. Thereafter, the obtained solution was first frozen (- 40 °C for 12h), and then freeze-dried (- 40 °C for 24h) to remove water to obtain injectable PHB microspheres.

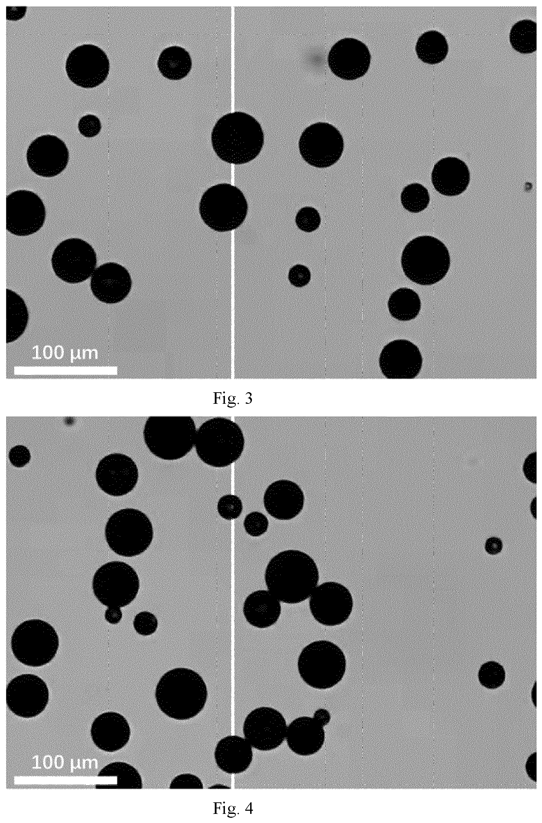

The yield of the obtained injectable PHB microspheres was 92%. The freeze-dried PHB microspheres were dissolved in water, and they can be dispersed in water upon proper mixing. After the water was air dried, the resultant was observed on an optical microscope. The results are shown in Figure 3. Figure 3 is the optical microscope picture of the injectable PHB microspheres obtained in Example 3. It can be seen that the obtained microspheres have good dispersion, which are spherical or ellipsoidal. The particle size is 2-55 µm as detected by particle size meter.

### Example 4

1g of PHBV (molecular weight 20-100 KDa, average molecular weight 40 KDa) was dissolved in 20mL of dichloromethane to prepare an organic solution with a final concentration of 5% (w/v), which was the oil phase. Hyaluronic acid (molecular weight 5-100 KDa, average molecular weight 50 KDa) was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 5h to remove the organic solvent. Thereafter, the obtained solution was first frozen (- 40 °C for 12h), and then freeze-dried (- 40 °C for 24h) to remove water to obtain injectable PHBV microspheres.

The yield of the obtained injectable PHBV microspheres was 90%. The freeze-dried PHBV microspheres were dissolved in water, and they can be dispersed in water upon proper mixing. After the water was air dried, the resultant was observed on an optical microscope. The results are shown in Figure 4. Figure 4 is the optical microscope picture of the injectable PHBV microspheres obtained in Example 4. It can be seen that the obtained microspheres have good dispersion, which are spherical or ellipsoidal. The particle size is 1-52 µm as detected by particle size meter.

### Example 5

1g of P34HB (molecular weight 10-30 KDa, average molecular weight 15 KDa) was dissolved in 20mL of dichloromethane to prepare an organic solution with a final concentration of 5% (w/v), which was the oil phase. Hyaluronic acid (molecular weight 5-100 KDa, average molecular weight 50 KDa) was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 5h to remove the organic solvent. Thereafter, the obtained solution was first frozen (- 40°C for 12h), and then freeze-dried (- 40°C for 24h) to remove water to obtain injectable P34HB microspheres.

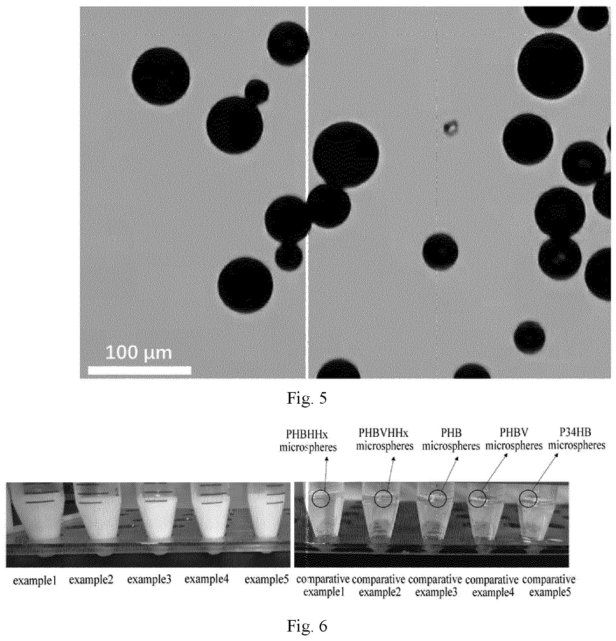

The yield of the obtained injectable P34HB microspheres was 90%. The freeze-dried P34HB microspheres were dissolved in water, and they can be dispersed in water upon proper mixing. After the water was air dried, the resultant was observed on an optical microscope. The results are shown in Figure 5. Figure 5 is the optical microscope picture of the injectable P34HB microspheres obtained in Example 5. It can be seen that the obtained microspheres have good dispersion, which are spherical or ellipsoidal. The particle size is 5-100 µm as detected by particle size meter, wherein, 68% of particles have a particle size of 1-60 µm. That is, most particles have a particle size of 60 µm or below.

### Example 6: Preparation of microspheres at different oil phase concentrations

0.5g, 1g and 2g of PHBHHx (molecular weight 20-100 KDa, average molecular weight 50 KDa) were dissolved in 20mL of dichloromethane, respectively, to prepare organic solutions with final concentrations of 2.5% (w/v), 5% (w/v) and 10% (w/v), which were oil phase. Hyaluronic acid (molecular weight 5-100 KDa, average molecular weight 50 KDa) was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 5h to remove the organic solvent. Thereafter, the obtained solution was first frozen (- 80°C for 6h), and then freeze-dried (- 40°C for 24h) to remove water to obtain injectable PHBHHx microspheres.

The yields of the obtained injectable PHBHHx microspheres were 90%, 93% and 89%, respectively. The freeze-dried PHBHHx microspheres were dissolved in water, and they can be dispersed in water upon proper mixing. After the water was air dried, the resultant was observed on an optical microscope. The results show that the obtained microspheres have good dispersion, which are spherical or ellipsoidal. As detected by particle size meter, the particle sizes are 0.4-20 µm, 1-50 µm, 1-80 µm, respectively (83% of microspheres have particle size of 1-60 µm).

### Example 7: Preparation of microspheres in different volume ratios of oil phase to aqueous phase

1g of PHBHHx (molecular weight 20-100 KDa, average molecular weight 50 KDa) was dissolved in 20mL of dichloromethane to prepare an organic solution with a final concentration of 5% (w/v), which was the oil phase. Hyaluronic acid (molecular weight 5-100 KDa, average molecular weight 50 KDa) was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratios of the two solutions were 1 : 10, 1 : 50 and 1 : 200, respectively. The stirring was continued using the magnetic stirrer at a speed of 400 rpm to form emulsions; The stirring was further continued at such speed for 5h to remove the organic solvent. Thereafter, the obtained solutions were first frozen (- 80°C for 6h), and then freeze-dried (- 40°C for 24h) to remove water to obtain injectable PHBHHx microspheres.

The yields of the obtained injectable PHBHHx microspheres were 71%, 93% and 94%, respectively. The freeze-dried PHBHHx microspheres were dissolved in water, and they can be dispersed in water upon proper mixing. After the water was air dried, the resultant was observed on an optical microscope. The results show that the obtained microspheres have good dispersion, which are spherical or ellipsoidal. As detected by particle size meter, the particle sizes are 1-110 µm (58% of particles have a size of 1-60 µm), 1-50 µm, 1-50 µm, respectively.

Example 8 Preparation of microspheres with different mixing mode and stirring speed 1g of PHBHHx (molecular weight 20-100 KDa, average molecular weight 50 KDa) was dissolved in 20mL of dichloromethane to prepare an organic solution with a final concentration of 5% (w/v), which was the oil phase. Hyaluronic acid (molecular weight 5-100 KDa, average molecular weight 50 KDa) was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using a magnetic stirrer (at 100 rpm, 400 rpm, 500 rpm, respectively) and a high-speed homogenizer (at 12000 rpm) respectively, to form emulsions; The stirring was further continued for 5h to remove the organic solvent. Thereafter, the obtained solutions were first frozen (- 80°C for 6h), and then freeze-dried (- 40°C for 24h) to remove water to obtain injectable PHBHHx microspheres.

The yields of the obtained injectable PHBHHx microspheres were 77%, 93%, 89% and 99%, respectively. The freeze-dried PHBHHx microspheres were dissolved in water, and they can be dispersed in water upon proper mixing. After the water was air dried, the resultant was observed on an optical microscope. The results show that the obtained microspheres have good dispersion, which are spherical or ellipsoidal. As detected by particle size meter, the particle sizes are 5-60 µm, 1-50 µm, 1-45 µm, 0.1-10 µM (particle size is basically 10 µm or below, only 5% of particles have a particle size of 1-60 µm. The overall particle size distribution is at a level of 0.1-10 µM), respectively. It is proved that when compared with the high-speed homogenization stirring method, stirring by using the magnetic stirrer at a proper speed (100-500rpm) in the present invention is more beneficial for obtaining a suitable particle size for human body.

### Example 9 Preparation of microspheres with different types of organic solvents in the oil phase

1g of PHBHHx (molecular weight 20-100 KDa, average molecular weight 50 KDa) was dissolved in 20mL of dichloromethane, chloroform and ethyl acetate, respectively, to prepare organic solutions with a final concentration of 5% (w/v), which were the oil phases. Hyaluronic acid (molecular weight 5-100 KDa, average molecular weight 50 KDa) was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form emulsions; The stirring was further continued at such speed for 5h to remove the organic solvent. Thereafter, the obtained solution was first frozen (- 80°C for 6h), and then freeze-dried (- 40°C for 24h) to remove water to obtain injectable PHBHHx microspheres.

The yields of the obtained injectable PHBHHx microspheres were 93%, 92%, and 89%, respectively. The freeze-dried PHBHHx microspheres were dissolved in water, and they can be dispersed in water upon proper mixing. After the water was air dried, the resultant was observed on an optical microscope. The results show that the obtained microspheres have good dispersion, which are spherical or ellipsoidal. As detected by particle size meter, the particle sizes are 1-52 µm, 1-50 µm, 1-55 µm, respectively.

### Example 10 Preparation of microspheres with different concentrations of the aqueous phase

1g of PHBHHx (molecular weight 20-100 KDa, average molecular weight 50 KDa) was dissolved in 20mL of dichloromethane to prepare an organic solution with a final concentration of 5% (w/v), which was the oil phases. Hyaluronic acid (molecular weight 10-200 KDa, average molecular weight 50 KDa) was dissolved in distilled water to prepare aqueous solutions with final concentrations of 0.01% (w/v), 0.5% (w/v) and 1% (w/v), respectively, which were the aqueous phases. The aqueous phases were stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form emulsions; The stirring was further continued at such speed for 5h to remove the organic solvent. Thereafter, the obtained solution was first frozen (- 80°C for 6h), and then freeze-dried (- 40°C for 24h) to remove water to obtain injectable PHBHHx microspheres.

The yields of the obtained injectable PHBHHx microspheres were 80%, 93%, and 87%, respectively. The freeze-dried PHBHHx microspheres were dissolved in water, and they can be dispersed in water upon proper mixing. After the water was air dried, the resultant was observed on an optical microscope. The results show that the obtained microspheres have good dispersion, which are spherical or ellipsoidal. As detected by particle size meter, the particle sizes are 3-50 µm, 1-50 µm, and 0.7-50 µm, respectively.

### Comparative example 1

1g of PHBHHx (molecular weight 20-100 KDa, average molecular weight 50 KDa) was dissolved in 20mL of dichloromethane to prepare an organic solution with a final concentration of 5% (w/v), which was the oil phase. Polyvinyl alcohol (PVA) with alcoholysis degree of 80% was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 5h to remove the organic solvent. Thereafter, the obtained solution was first frozen (- 80°C for 6h), and then freeze-dried (- 40°C for 24h) to remove water to obtain PHBHHx microspheres.

### Comparative example 2

1g of PHBVHHx (molecular weight 20-100 KDa, average molecular weight 50 KDa) was dissolved in 20mL of dichloromethane to prepare an organic solution with a final concentration of 5% (w/v), which was the oil phase. Polyvinyl alcohol (PVA) with alcoholysis degree of 80% was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using a magnetic stirrer at a speed of 400 rpm to form an emulsion; The stirring was further continued at such speed for 5h to remove the organic solvent. Thereafter, the obtained solution was first frozen (- 80°C for 6h), and then freeze-dried (- 40°C for 24h) to remove water to obtain PHBVHHx microspheres.

### Comparative example 3

1g of PHB (molecular weight 20-100 KDa, average molecular weight 80 KDa) was dissolved in 20mL of dichloromethane to prepare an organic solution with a final concentration of 5% (w/v), which was the oil phase. Polyvinyl alcohol (PVA) with alcoholysis degree of 80% was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 5h to remove the organic solvent. Thereafter, the obtained solution was first frozen (- 80°C for 6h), and then freeze-dried (- 40°C for 24h) to remove water to obtain PHB microspheres.

### Comparative example 4

1g of PHBV (molecular weight 20-100 KDa, average molecular weight 40 KDa) was dissolved in 20mL of dichloromethane to prepare an organic solution with a final concentration of 5% (w/v), which was the oil phase. Polyvinyl alcohol (PVA) with alcoholysis degree of 80% was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 5h to remove the organic solvent. Thereafter, the obtained solution was first frozen (- 80°C for 6h), and then freeze-dried (- 40°C for 24h) to remove water to obtain PHBV microspheres.

### Comparative example 5

1g of P34HB (molecular weight 10-30 KDa, average molecular weight 15 KDa) was dissolved in 20mL of dichloromethane to prepare an organic solution with a final concentration of 5% (w/v), which was the oil phase. Polyvinyl alcohol (PVA) with alcoholysis degree of 80% was dissolved in distilled water to prepare an aqueous solution with a final concentration of 0.5% (w/v), which was the aqueous phase. The aqueous phase was stirred using a magnetic stirrer at a speed of 400 rpm, meanwhile, the oil phase was slowly added dropwise into the aqueous phase with stirring. The volume ratio of the two solutions was 1 : 50. The stirring was continued using the magnetic stirrer at a speed of 400 rpm to form an emulsion; The stirring was further continued at such speed for 5h to remove the organic solvent. Thereafter, the obtained solution was first frozen (- 80°C for 6h), and then freeze-dried (- 40°C for 24h) to remove water to obtain P34HB microspheres.

### Example 11: Stability test of microspheres

0.01g of microspheres prepared in examples 1 to 5 and comparative examples 1 to 5 were weighed, dispersed in 1mL of distilled water, and placed in 5mL glass bottles, respectively. After rapid mixing, they were let stand.

After standing for 30 minutes, the dispersion of the microspheres in water was observed. The results are shown in Figure 6. Figure 6 shows the dispersion stability of the microspheres obtained in examples 1 to 5 and comparative examples 1 to 5 (10 containers from left to right correspond to samples in Examples 1 to 5 and comparative examples 1 to 5, respectively). It can be seen that the PHBHHx microspheres, PHBVHHx microspheres, PHB microspheres, PHBV microspheres and P34HB microspheres in examples 1 to 5 had good dispersibility, showing no layering, and the microspheres were uniformly dispersed in water; the PHBHHx microspheres, PHBVHHx microspheres, PHB microspheres, PHBV microspheres and P34HB microspheres in comparative examples 1 to 5 showed poor water dispersibility, and serious layering, and the microspheres floated in the upper layer of the water. Thus, it is proved that compared with the case in which the polyvinyl alcohol aqueous solution is used as the aqueous phase material, the present invention significantly improves the dispersion and stability of microspheres by using hyaluronic acid aqueous solution as the aqueous phase material.

### Example 12: Absorbability test of microspheres

A disposable 1mL syringe with a needle with an inner diameter of 0.5mm and a length of 19.7mm (that is, the commonly used 1mL syringe gun head) was used as the injection rate detection device, and 1mL of microsphere dispersion (the dry weight of microsphere was 0.1g, recorded as M0) was used as the experimental sample. 0.1g of microspheres prepared in examples 1 to 5 and comparative examples 1 to 5 were weighed, dispersed in 1mL distilled water, and placed in glass bottles, respectively. After rapid mixing uniformly, microsphere dispersions were formed. 1mL of dispersion was drawn by 1mL syringe, and the microspheres drawn in the syringe were freeze-dried respectively; The percentage of the microsphere mass (M1) drawn in the syringe in the total microsphere mass (M0) was the absorbability of microspheres (M1/M0) × 100%_{∘}

The results show that the absorbabilities of the PHBHHx microspheres, PHBVHHx microspheres, PHB microspheres, PHBV microspheres and P34HB microspheres in examples 1 to 5 were 95%, 93%, 91%, 90% and 85% respectively. The absorbabilities of the PHBHHx microspheres, PHBVHHx microspheres, PHB microspheres, PHBV microspheres and P34HB microspheres in comparative examples 1 to 5 were 10%, 13%, 11%, 9% and 6% respectively. It can be seen that the absorbabilities of microspheres in examples 1 to 5 were much higher than those in comparative examples 1 to 5. Thus, it is proved that compared with the case in which the polyvinyl alcohol aqueous solution is used as the aqueous phase material, the present invention significantly improves the absorbability of microspheres by using hyaluronic acid aqueous solution as the aqueous phase material.

### Example 13: Injectability test of microspheres

A disposable 1mL syringe with a needle with an inner diameter of 0.5mm and a length of 19.7mm (that is, the commonly used 1mL syringe gun head) was used as the injection rate detection device, and 1mL of microsphere dispersion (the dry weight of microsphere was 0.1g, recorded as M0) was used as the experimental sample. 0.1g of microspheres prepared in examples 1 to 5 and comparative examples 1 to 5 were weighed, dispersed in 1mL distilled water, and placed in glass bottles, respectively. After rapid mixing uniformly, microsphere dispersions were formed. The dispersions were completely injected into a 1mL syringe, after pushing the syringe, the dispersions injected out were collected and freeze-dried, respectively, and percentage of the microsphere mass (M2) of the dispersions injected out in the total microsphere mass (M0) was calculated as the injection rate of microspheres: (M2/M0) × 100%.

The results show that the injection rates of the PHBHHx microspheres, PHBVHHx microspheres, PHB microspheres, PHBV microspheres and P34HB microspheres in examples 1 to 5 were 91%, 92%, 90%, 90% and 88%, respectively. The injection rates of the PHBHHx microspheres, PHBVHHx microspheres, PHB microspheres, PHBV microspheres and P34HB microspheres in comparative examples 1 to 5 were 7%, 11%, 13%, 8% and 4%, respectively. It can be seen that the injection rates of microspheres in examples 1 to 5 were much higher than those in comparative examples 1 to 5. Thus, it is proved that compared with the case in which the polyvinyl alcohol aqueous solution is used as the aqueous phase material, the present invention significantly improves the injectability of microspheres by using hyaluronic acid aqueous solution as the aqueous phase material.

The above experimental results show that the preparation method provided by the invention has the following beneficial effects: ① the yield of microspheres is high: the yield of microspheres is 70% or more. ② Good dispersibility: The PHA microspheres prepared by the invention can be dispersed in water upon proper mixing. After standing, the microspheres show good dispersibility in water and no layering, they neither float on the upper layer of water nor settle on the bottom of water, rather they are evenly dispersed in water to form a uniform dispersion. The microspheres are observed for their morphology after the above dispersion is air dried and the results show that the microspheres are complete in morphology, independent of each other, and there is no agglomeration phenomenon. All or most of the microspheres have particle size of 60 µm or less, and they can meet the injection requirements. ③ Good absorbability: the absorption rate of the microsphere reaches 85% or more by using a syringe to draw the above dispersion, showing excellent absorbability. ④ Good injectability: pushing a syringe and collecting the dispersion injected out, calculating the injection rate of the microspheres, and the results show that the injection rate of the microspheres is 88% or more, showing excellent injectability.

In the present application, the principle and embodiments of the invention are illustrated through specific examples and the above examples are only used to help those skilled in the art to understand the method and core idea of the invention, including the best embodiment, and also enable any person skilled in the art to practice the invention, including manufacturing and using any device or system, and implementing any combined methods. The protection scope of the invention is limited by the claims.

## Claims

1. A preparation method of polyhydroxyalkanoate (PHA) microspheres, comprises the following steps:
a) dissolving a PHA material in an organic solvent to obtain an oil phase;
the PHA material has a molecular weight of 10-100KDa;
the mass concentration of the PHA material in the oil phase is 2.5%-10%;
b) dissolving hyaluronic acid in water to obtain an aqueous phase;
the hyaluronic acid has a molecular weight of 5-500KDa;
the mass concentration of hyaluronic acid in the aqueous phase is 0.01%-1%;
c) adding the oil phase to the aqueous phase dropwise with stirring, thereafter evaporating off the organic solvent under continuous stirring, and then carrying out solid-liquid separation and drying to obtain PHA microspheres;
the order of steps a) and b) is not limited.

2. The preparation method according to claim 1, wherein in step c), the volume ratio of the oil phase to the aqueous phase is 1 : (10-200).

3. The preparation method according to claim 1, wherein the organic solvent is selected from one or more of dichloromethane, chloroform and ethyl acetate.

4. The preparation method according to claim 1, wherein the average molecular weight of the PHA material is 15-80KDa.

5. The preparation method according to claim 1, wherein the PHA material is PHBHHx, with a molecular weight of 20-100 KDa and an average molecular weight of 50 KDa; or
the PHA material is PHBVHHx, with a molecular weight of 20-100 KDa and an average molecular weight of 50 KDa; or
the PHA material is PHB, with a molecular weight of 20-100 KDa and an average molecular weight of 80 KDa; or
the PHA material is PHBV, with a molecular weight of 20-100 KDa and an average molecular weight of 40 KDa; or
the PHA material is P34HB, with a molecular weight of 10-30 KDa and an average molecular weight of 15 KDa.

6. The preparation method according to claim 1, wherein the average molecular weight of the hyaluronic acid is 50-300 KDa.

7. The preparation method according to claim 1, wherein the hyaluronic acid has a molecular weight of 5-100 KDa, and an average molecular weight of 50 KDa; or
the hyaluronic acid has a molecular weight of 10-200 KDa, and an average molecular weight of 50 KDa; or
the hyaluronic acid has a molecular weight of 100-500 KDa, and an average molecular weight of 300 KDa.

8. The preparation method according to claim 1, wherein the stirring rate is 100~500 rpm.

9. The preparation method according to claim 1, wherein the stirring is performed by using a magnetic stirrer;
after the addition of the oil phase, the stirring time is ≥ 4h.

10. The preparation method according to claim 9, wherein after the addition of the oil phase, the stirring time is 4h-6h.

11. The preparation method according to claim 1, wherein the stirring temperature is 10~37 °C.

12. The preparation method according to claim 1, wherein the drying is performed by freezing first and then freeze-drying;
the freezing temperature is -80 ~ -20 °C, and the freeze-drying temperature is -60 ~ -40 °C.

13. A polyhydroxyalkanoate microsphere prepared by the preparation method of any one of claims 1 to 12.

14. The polyhydroxyalkanoate microsphere according to claim 13, wherein the particle size of the polyhydroxyalkanoate microsphere is 60 µm or less; and/or the absorption rate of the polyhydroxyalkanoate microsphere is 85% or above; and/or the injection rate of the polyhydroxyalkanoate microspheres is 88% or above.

## Patentansprüche

1. Verfahren zur Herstellung von Polyhydroxyalkanoat (PHA)-Mikrokügelchen, umfassend die folgenden Schritte:
a) Auflösen eines PHA-Materials in einem organischen Lösungsmittel, um eine Ölphase zu erhalten;
das PHA-Material hat ein Molekulargewicht von 10-100 kDa;
die Massenkonzentration des PHA-Materials in der Ölphase beträgt 2,5 % bis 10 %;
b) Auflösen von Hyaluronsäure in Wasser, um eine wässrige Phase zu erhalten;
die Hyaluronsäure hat ein Molekulargewicht von 5-500 kDa;
die Massenkonzentration der Hyaluronsäure in der wässrigen Phase beträgt 0,01 % bis 1 %;
c) Zugabe der Ölphase zur wässrigen Phase tropfenweise unter Rühren, anschließendes Abdampfen des organischen Lösungsmittels unter ständigem Rühren und anschließende Fest-Flüssig-Trennung und Trocknung, um PHA-Mikrokügelchen zu erhalten;
die Reihenfolge der Schritte a) und b) ist nicht festgelegt.

2. Herstellungsverfahren nach Anspruch 1, wobei in Schritt c) das Volumenverhältnis der Ölphase zur wässrigen Phase 1: (10-200) beträgt.

3. Herstellungsverfahren nach Anspruch 1, wobei das organische Lösungsmittel aus einem oder mehreren der folgenden ausgewählt ist: Dichlormethan, Chloroform und Ethylacetat.

4. Herstellungsverfahren nach Anspruch 1, wobei das durchschnittliche Molekulargewicht des PHA-Materials 15-80 kDa beträgt.

5. Herstellungsverfahren nach Anspruch 1, wobei das PHA-Material PHBHHx mit einem Molekulargewicht von 20-100 kDa und einem durchschnittlichen Molekulargewicht von 50 kDa ist; oder
das PHA-Material PHBVHHx ist, mit einem Molekulargewicht von 20-100 kDa und einem durchschnittlichen Molekulargewicht von 50 kDa; oder
das PHA-Material PHB ist, mit einem Molekulargewicht von 20-100 kDa und einem durchschnittlichen Molekulargewicht von 80 kDa; oder
das PHA-Material ist PHBV mit einem Molekulargewicht von 20-100 kDa und einem durchschnittlichen Molekulargewicht von 40 kDa; oder
das PHA-Material P34HB ist, mit einem Molekulargewicht von 10-30 kDa und einem durchschnittlichen Molekulargewicht von 15 kDa.

6. Herstellungsverfahren nach Anspruch 1, wobei das durchschnittliche Molekulargewicht der Hyaluronsäure 50-300 kDa beträgt.

7. Herstellungsverfahren nach Anspruch 1, wobei die Hyaluronsäure ein Molekulargewicht von 5-100 kDa und ein durchschnittliches Molekulargewicht von 50 kDa aufweist; oder
die Hyaluronsäure ein Molekulargewicht von 10-200 kDa und ein durchschnittliches Molekulargewicht von 50 kDa aufweist; oder
die Hyaluronsäure ein Molekulargewicht von 100-500 kDa und ein durchschnittliches Molekulargewicht von 300 kDa aufweist.

8. Herstellungsverfahren nach Anspruch 1, wobei die Rührgeschwindigkeit 100~500 U/min beträgt.

9. Herstellungsverfahren nach Anspruch 1, wobei das Rühren unter Verwendung eines Magnetrührers durchgeführt wird;
nach Zugabe der Ölphase beträgt die Rührzeit ≥ 4 h.

10. Herstellungsverfahren nach Anspruch 9, wobei nach Zugabe der Ölphase die Rührzeit 4-6 Stunden beträgt.

11. Herstellungsverfahren nach Anspruch 1, wobei die Rührtemperatur 10 ~ 37 °C beträgt.

12. Herstellungsverfahren nach Anspruch 1, wobei das Trocknen durch vorheriges Einfrieren und anschließendes Gefriertrocknen erfolgt;
die Gefriertemperatur -80 ~ -20 °C beträgt und die Gefriertrocknungstemperatur -60 ~ -40 °C beträgt.

13. Polyhydroxyalkanoat-Mikrokügelchen, hergestellt durch das Herstellungsverfahren nach einem der Ansprüche 1 bis 12.

14. Polyhydroxyalkanoat-Mikrokügelchen nach Anspruch 13, wobei die Partikelgröße der Polyhydroxyalkanoat-Mikrokügelchen 60 µm oder weniger beträgt; und/oder
die Absorptionsrate der Polyhydroxyalkanoat-Mikrokügelchen 85 % oder mehr beträgt; und/oder
die Injektionsrate der Polyhydroxyalkanoat-Mikrokügelchen 88 % oder mehr beträgt.

## Revendications

1. Procédé de préparation de microsphères de polyhydroxyalcanoate (PHA), qui comprend les étapes suivantes :
a) dissolution d'un matériau PHA dans un solvant organique afin d'obtenir une phase huileuse ;
le matériau PHA a une masse moléculaire de 10 à 100 KDa ;
la concentration massique du matériau PHA dans la phase huileuse va de 2,5 % à 10 % ;
b) dissolution d'acide hyaluronique dans de l'eau afin d'obtenir une phase aqueuse ;
l'acide hyaluronique a une masse moléculaire de 5 à 500 KDa ;
la concentration massique d'acide hyaluronique dans la phase aqueuse va de 0,01 % à 1 % ;
c) ajout goutte à goutte et avec agitation de la phase huileuse dans la phase aqueuse, puis élimination du solvant organique par évaporation sous agitation continue, puis réalisation d'une séparation solide-liquide et séchage afin d'obtenir les microsphères de PHA ;
l'ordre des étapes a) et b) n'est pas limité.

2. Procédé de préparation selon la revendication 1, dans lequel à l'étape c), le rapport volumique de la phase huileuse à la phase aqueuse est de 1:(10 à 200).

3. Procédé de préparation selon la revendication 1, dans lequel le solvant organique est choisi entre un ou plusieurs solvants parmi le dichlorométhane, le chloroforme et l'acétate d'éthyle.

4. Procédé de préparation selon la revendication 1, dans lequel la masse moléculaire moyenne du matériau PHA va de 15 à 80 KDa.

5. Procédé de préparation selon la revendication 1, dans lequel le matériau PHA est le PHBHHx, avec une masse moléculaire de 20 à 100 KDa et une masse moléculaire moyenne de 50 KDa ; ou
le matériau PHA est le PHBVHHx, avec une masse moléculaire de 20 à 100 KDa et une masse moléculaire moyenne de 50 KDa ; ou
le matériau PHA est le PHB, avec une masse moléculaire de 20 à 100 KDa et une masse moléculaire moyenne de 80 KDa ; ou
le matériau PHA est le PHBV, avec une masse moléculaire de 20 à 100 KDa et une masse moléculaire moyenne de 40 KDa ; ou
le matériau PHA est le P34HB, avec une masse moléculaire de 10 à 30 KDa et une masse moléculaire moyenne de 15 KDa.

6. Procédé de préparation selon la revendication 1, dans lequel la masse moléculaire moyenne de l'acide hyaluronique va de 50 à 300 KDa.

7. Procédé de préparation selon la revendication 1, dans lequel l'acide hyaluronique a une masse moléculaire de 5 à 100 KDa et une masse moléculaire moyenne de 50 KDa ; ou
l'acide hyaluronique a une masse moléculaire de 10 à 200 KDa et une masse moléculaire moyenne de 50 KDa ; ou
l'acide hyaluronique a une masse moléculaire de 100 à 500 KDa et une masse moléculaire moyenne de 300 KDa.

8. Procédé de préparation selon la revendication 1, dans lequel la vitesse d'agitation est de 100 à 500 tours/minute.

9. Procédé de préparation selon la revendication 1, dans lequel l'agitation est effectuée à l'aide d'un agitateur magnétique ;
après l'ajout de la phase huileuse, la durée d'agitation est ≥ 4 h.

10. Procédé de préparation selon la revendication 9, dans lequel après l'ajout de la phase huileuse, la durée d'agitation va de 4 h à 6 h.

11. Procédé de préparation selon la revendication 1, dans lequel la température d'agitation est de 10 à 37 °C.

12. Procédé de préparation selon la revendication 1, dans lequel le séchage est effectué d'abord par congélation, puis par lyophilisation ;
la température de congélation est de -80 à -20 °C, et la température de lyophilisation est de -60 à -40 °C.

13. Microsphère de polyhydroxyalcanoate préparée par le procédé de préparation selon l'une quelconque des revendications 1 à 12.

14. Microsphère de polyhydroxyalcanoate selon la revendication 13, dans laquelle la taille de particule de la microsphère de polyhydroxyalcanoate est de 60 µm ou moins ; et/ou
le taux d'absorption de la microsphère de polyhydroxyalcanoate est de 85 % ou plus ; et/ou
le taux d'injection de la microsphère de polyhydroxyalcanoate est de 88 % ou plus.
